# FASCICULE DE BREVET EUROPEEN

(11) **EP 1 708 768 B1**
(45) Date de publication et mention de la délivrance du brevet: **20.06.2018**
(21) Numéro de dépôt: 05717394.0
(22) Date de dépôt: 11.01.2005
(51) Int. Cl.: A61M 5/30, A61M 5/31

(54) **SERINGUE SANS AIGUILLE AVEC UN INJECTEUR RECEPTACLE AMORTISSANT**
NADELLOSE SPRITZE MIT EINER DÄMPFENDEN INJEKTOR-AUFNAHME
NEEDLELESS SYRINGE PROVIDED WITH A DAMPING INJECTOR-RECEPTACLE

(30) Priorité: 27.01.2004 FR 0400721
(43) Date de publication de la demande: 11.10.2006
(73) Titulaire: Crossject, 21000 Dijon (FR)
(72) Inventeur: ALEXANDRE, Patrick, F-70100 Gray (FR); BAUD, Georges, F-83260 La Crau (FR); BROUQUIERES, Bernard, F-83100 Toulon (FR); D'EMMANUELLE, Laurent, F-83200 Toulon (FR); ROLLER, Denis, F-91590 La Ferte Alais (FR)
(74) Mandataire: Gaillarde, Frédéric F. Ch.
(86) Numéro de dépôt international: PCT/FR2005/000051
(87) Numéro de publication internationale: WO 2005/082438

(56) Documents cités:
- WO-A-01/58512
- WO-A-01/89614
- WO-A-01/97884
- DE-A- 10 211 473
- FR-A- 2 815 544

## Description

La présente invention est dans le domaine des seringues sans aiguille, pré-remplies et jetables ; de telles seringues sont utilisées pour les injections intradermiques, sous-cutanées et intramusculaires, de principe actif liquide à usage thérapeutique en médecine humaine ou vétérinaire.

Un premier impératif pour des seringues préremplies est celui de la compatibilité à long terme, trois ans en général, entre le principe actif liquide et le réservoir qui le contient. Un autre impératif, lié au procédé de pré-remplissage, est d'avoir un réservoir transparent pour faire les contrôles réglementaires du remplissage correct du réservoir avant son montage dans la seringue. Ces impératifs conduisent à la réalisation de réservoir essentiellement transparent et en matériau compatible avec le principe actif pour la durée souhaitée : c'est en général du verre à usage pharmaceutique : verre de type I ou II.

La phase initiale de l'injection est critique pour la pénétration dans la peau du jet ou des jets de liquide, suivant que la seringue a un ou plusieurs conduits d'injection. Cette dernière configuration étant favorable pour réduire la douleur. La biodisponibilité finale dépend de la bonne réalisation de cette phase initiale, elle suppose une mise en vitesse rapide du liquide dans les conduits d'injection sans les saccades multiples des jets quand il y a un coup de bélier trop important pour réaliser cette mise en vitesse rapide. La demande de brevet WO 01/58512 décrit une seringue sans aiguille comportant un réservoir fermé par des obturateurs amont et aval déplaçables emprisonnant un principe actif liquide ; ledit réservoir est initialement isolé d'un injecteur ou réceptacle qui comporte au moins deux conduits d'injection situés sur sa face latérale extérieure et un alésage central borgne dans lequel va venir se loger l'obturateur aval de façon à dégager les entrées des conduits d'injection lors du déplacement de l'ensemble mobile comprenant l'ensemble obturateur aval - principe actif - obturateur amont par l'action d'un dispositif moteur pour réaliser l'injection. Le problème à résoudre avec ce type de seringue est l'amortissement de l'impact de l'ensemble mobile quand l'obturateur aval arrive au contact du fond de l'alésage du réceptacle et aussi celui d'éviter les rebonds dudit obturateur aval après cet impact.

Le document DE 10211473 décrit une seringue sans aiguille comportant un corps logeant un réservoir
cylindrique fermé par un obturateur amont déplaçable et un obturateur aval déplaçable emprisonnant un principe actif et comportant à l'aval un réceptacle avec au moins un conduit d'injection, ledit réceptacle étant en appui sur le réservoir et comprenant un alésage, ayant une paroi latérale qui comporte au moins une protubérance, et dans lequel se loge l'obturateur aval quand il est amené au contact du fond de l'alésage dudit réceptacle par le fonctionnement d'un moyen moteur déplaçant l'ensemble obturateur amont-liquide-obturateur aval.

Dans ce document, la protubérance est réalisée par un épaississement progressif vers l'intérieur de la paroi de l'alésage depuis l'ouverture amont de l'alésage vers son extrémité aval. Cette seringue est satisfaisante du point de vue de l'amortissement de l'impact car l'obturateur aval s'écrase de plus en plus au fur et à mesure qu'il s'enfonce dans l'alésage, mais la protubérance n'a aucun effet anti-rebond.

Selon l'invention on propose une seringue comme définie dans la revendication 1 et dans laquelle au moins une protubérance fait striction par rapport à l'ouverture amont de l'alésage et est configurée pour bloquer, dans l'alésage, l'obturateur aval quand il y sera engagé.

Ainsi dans le sens de l'amortissement de l'impact, l'obturateur aval s'écrase de façon élastique sur la face amont de la striction, et passe progressivement en aval de la striction de sorte qu'en cas de rebond la partie de l'obturateur aval qui est passée à travers la striction prend appui sur la face aval de la striction et s'oppose donc au rebond.

Remarquons que l'alésage central peut être borgne avec un fond sensiblement plat ou comporter au moins un picot sur lequel va venir se déformer et se déchirer l'obturateur aval. L'alésage central peut aussi comporter, en son fond, au moins un orifice. De plus cet orifice peut être calibré de façon à limiter le débit d'air refoulé par l'obturateur aval et participer ainsi à un freinage pneumatique de l'ensemble mobile.

Dans cette demande est désignée avec le qualificatif aval toute pièce proche du site d'injection ou toute partie de pièce dirigée vers ce site d'injection, ce site est la peau du patient. A contrario le qualificatif amont sera utilisé pour toute pièce éloignée du site d'injection ou toute partie de pièce dirigée à l'opposé de ce site. Ainsi le réceptacle comporte une face aval dirigée vers la peau du patient et une face amont qui lui est opposée et est en appui sur le réservoir ; ces faces aval et amont sont reliées par une face latérale.

Dans cette invention, par principe actif liquide, ou médicament, nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée, ainsi que la forme du conduit, pour éviter les bouchages.

Le réservoir, essentiellement cylindrique, est en verre de type I ou de type II ; mais il peut être en tout autre matériau transparent et compatible avec le principe actif. Les faces amont et aval du réservoir sont essentiellement planes, les plans les contenant étant perpendiculaires à l'axe de symétrie du réservoir. Les faces amont et aval sont en appui respectivement avec le corps de la seringue et le réceptacle. Les faces d'appui de ces deux pièces comportent des joints dont les caractéristiques seront précisées par la suite.

Un conduit d'injection traverse toute la hauteur du réceptacle depuis la face amont jusqu'à la face aval. Les conduits d'injection, quand il y en a au moins deux, sont dits périphériques car ils sont disposés dans le réceptacle autour de l'alésage central. Ils ne communiquent avec ledit alésage central que par des entrées décrites par la suite. Le conduit d'injection a une section variable de l'amont à l'aval d'une part pour des raisons liées à sa réalisation et d'autre part pour obtenir un jet suffisamment fin et rapide pour pénétrer, à la profondeur souhaitée, dans la peau du patient. En général les conduits d'injection sont identiques, équirépartis autour de l'alésage central borgne et ont des axes parallèles à l'axe de réceptacle, mais ils peuvent aussi être différents. Quand il y a peu de principe actif à injecter, un seul conduit d'injection est suffisant.

Le moyen moteur qui va agir sur l'obturateur amont et tout l'ensemble mobilepeut être un moteur mécanique : détente d'un ressort comprimé ou du type pneumatique : détente de gaz comprimé, ou pyrotechnique : détente de gaz de combustion.

Le fonctionnement de la seringue est le suivant : le moyen moteur va agir sur l'obturateur amont et déplacer l'ensemble "obturateur amont - liquide - obturateur aval" puisque le liquide est incompressible. L'obturateur aval se déplace et se loge dans l'alésage central du réceptacle jusqu'au contact avec le fond dudit alésage. Le volume de cet alésage est tel que lorsque l'obturateur aval est au contact du fond dudit alésage les entrées des conduits d'injection, sur la périphérie de l'alésage du réceptacle, sont dégagées ; le liquide y est refoulé et est injecté par le mouvement de l'obturateur amont qui se poursuit jusqu'à la vidange du réservoir : l'obturateur amont est alors au contact de l'obturateur aval.

L'entrée d'un conduit d'injection, située sur la face amont du réceptacle comprend un lamage positionné et préférentiellement centré sur le conduit d'injection et un canal radial reliant ledit lamage à l'alésage central dudit réceptacle.

Avantageusement, la protubérance circulaire faisant striction de l'alésage est réalisée par la succession de deux parties tronconiques lorsqu'on pénètre dans l'alésage en suivant le déplacement de l'obturateur aval. Le tronc de cône situé à l'amont est convergent, il se raccorde à un tronc de cône divergent soit jusqu'à fond du réceptacle soit à une portion cylindrique se raccordant ensuite au fond du réceptacle. La partie tronconique convergente freine l'obturateur aval, puis la partie tronconique divergente qui lui succède va avoir pour rôle de bloquer, dans l'alésage, l'obturateur aval quand il y sera engagé.

Dans un deuxième mode de réalisation la striction est réalisée par la superposition, sur la hauteur totale ou partielle de l'alésage central de plusieurs protubérances circulaires, telles que précédemment décrites. Par exemple par une succession de plusieurs tronc de cônes alternativement convergents puis divergents. Dans une réalisation préférée les troncs de cône divergents peuvent être réduits à un brusque élargissement jusqu'au diamètre d'entrée de l'alésage ce qui va donner un profil en dents de scie aux protubérances de l'alésage.

Dans un troisième mode de réalisation la protubérance peut être une saillie hélicoïdale en forme de taraudage s'enroulant sur la paroi latérale de l'alésage central, le filet ayant une section appropriée.

Dans un quatrième mode de réalisation les protubérances sont des saillies le long d'une génératrice de la paroi de l'alésage central borgne. Préférentiellement lesdites saillies sont équiréparties tout autour de l'alésage et avantageusement ces saillies sont réalisées au droit des zones où se trouvent les conduits d'injection. Lesdites saillies ont soit une forme ovoïde de godron, soit sont crantées, plusieurs petites saillies en ergot se succèdent le long de la génératrice. D'une certaine façon ces protubérances en saillies peuvent être des parties des protubérances précédemment décrites qui alors ne sont pas entièrement circulaires mais sont seulement des secteurs autour d'une génératrice de l'alésage.

La présente invention appliquée à une seringue pré-remplie et à usage unique a l'avantage de permettre de séparer, dans le dispositif, deux parties. Une partie qui sera dite partie pharmaceutique comprenant le corps et le réservoir avec les obturateurs déplaçables amont et aval et éventuellement l'injecteur-réceptacle : ce sous-ensemble pourra être traité dans les conditions de l'industrie pharmaceutique notamment en ce qui concerne la stérilisation et l'asepsie. Ce sous-ensemble sera intégré au reste de la seringue, dont les éléments ont été assemblés par ailleurs, cet assemblage se faisant dans les conditions moins sévères que celles liées à l'industrie pharmaceutique.

Lorsque l'obturateur aval est logé dans l'alésage du réceptacle la seringue devient très difficilement réutilisable. Cette disposition a donc aussi l'avantage d'empêcher des réutilisations de ladite seringue à des fins différentes de l'utilisation thérapeutique initiale.

Enfin cette configuration présente l'avantage d'éviter des fuites éventuelles de liquide par les conduits d'injection avant la réalisation de l'injection. En effet l'agitation du dispositif est fréquemment réalisée, voire préconisée pour examiner la turbidité du liquide ou homogénéiser le mélange lorsque le liquide comporte des particules en suspension. Le fait que le principe actif soit isolé, avant injection, réalise une protection ultime vis à vis de ce risque de perte.

Ci-dessous l'invention est exposée en détail à l'aide de figures représentant différentes réalisations particulières de l'invention.

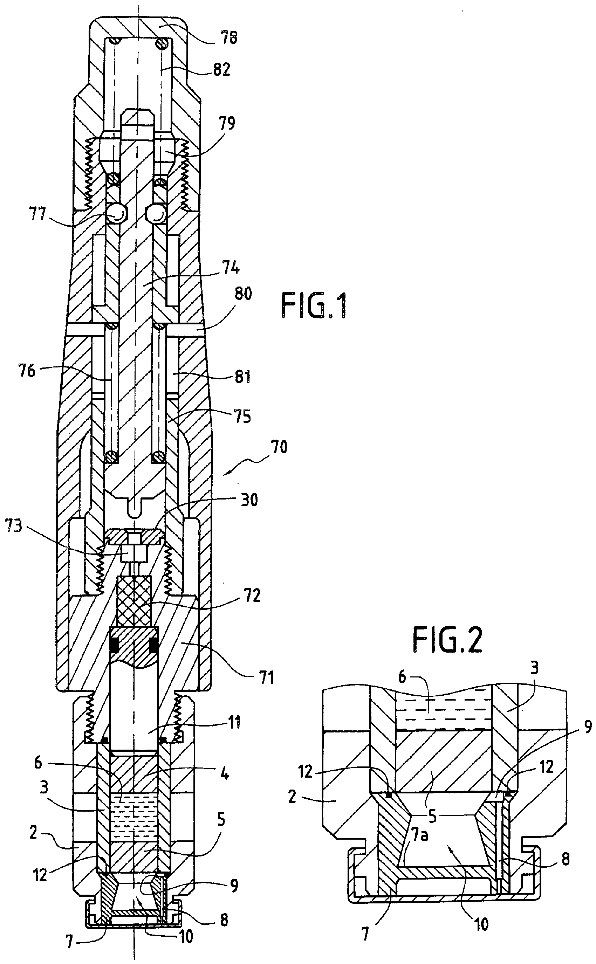

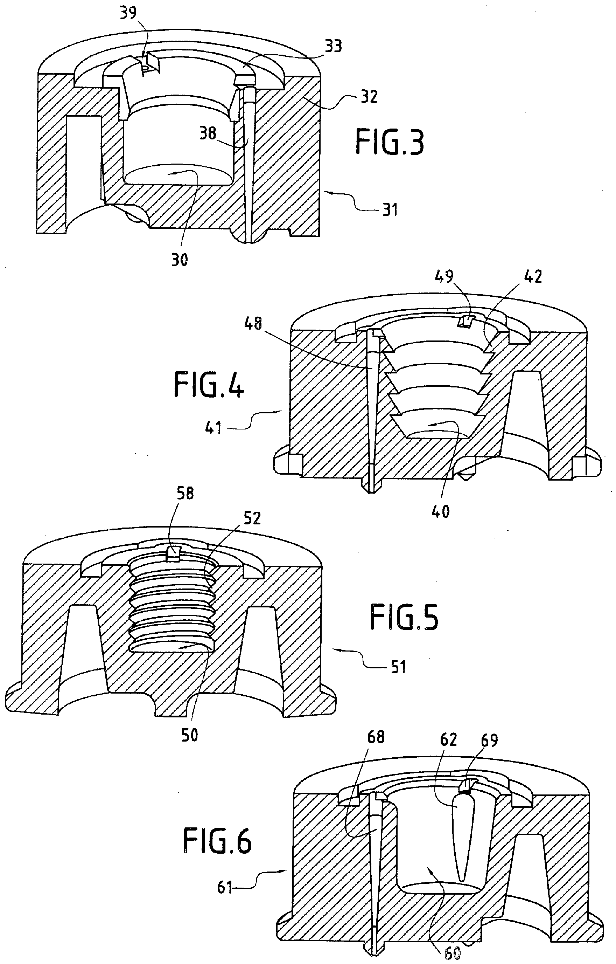

La figure 1 représente une coupe longitudinale d'une seringue selon une première réalisation de l'invention. La figure 2 est un agrandissement de la partie aval de ladite seringue. La figure 3 représente en perspective partiellement coupée un autre exemple de réalisation de l'invention selon le premier mode mais ici par l'assemblage de deux parties ou pièces. La figure 4 schématise en perspective coupée un réceptacle réalisé selon un second mode par la superposition de plusieurs strictions circulaires sur toute la hauteur de l'alésage. La figure 5 représente en perspective une réalisation de l'invention selon un troisième mode par un alésage taraudé ; la figure 6 présente de même un quatrième mode de réalisation de l'invention : les protubérances étant des saillies ovoïdes selon des génératrices latérales de l'alésage.

La figure 1 représente en coupe longitudinale partielle une seringue selon l'invention, elle est représentée verticale, le système d'injection dirigé vers le bas qui sera l'aval.

La seringue 1 comporte un corps 2 dans lequel est logé un réservoir 3 contenant le principe actif liquide 6. A l'extrémité aval du corps 2 est placé un réceptacle 7 comportant, par exemple, trois conduits d'injection tels que le conduit 8. Le système d'injection est recouvert d'une protection extérieure pour assurer l'asepsie de la seringue : cette protection comprend une membrane d'élastomère appliquée sur la face extérieure de l'injecteur par un opercule métallique fin, serti autour de cette extrémité de la seringue. Cette protection sera retirée avant l'injection. A son extrémité opposée, le corps 2 de la seringue est fixé à un moyen moteur 70 qui, dans cet exemple, est un générateur pyrotechnique de gaz, il sera décrit par la suite. Le réservoir 3 est en appui sur le corps 71 du moteur 70, l'étanchéité est assurée par un joint torique circulaire.

Le corps 2 de la seringue comporte deux fenêtres diamétralement opposées pour la visualisation du principe actif contenu dans le réservoir 3 : ce sont simplement deux ouvertures oblongues dans le corps. A l'aval du corps 2 de la seringue est emmanché, dans un alésage de forme appropriée, un réceptacle 7 qui sera décrit par la suite. En appui sur ce réceptacle 7 et centré dans l'aval du corps 2 est positionné un réservoir 3 de verre ; ce réservoir est un tube. En amont le corps 2 de la seringue reçoit le corps 71 du moyen moteur qui se centre autour de l'autre extrémité du réservoir. Le réservoir 3 est essentiellement un tube fermé à ses deux extrémités par des obturateurs déplaçables amont 4 et aval 5 ; ces obturateurs sont préférentiellement des bouchons-pistons habituellement utilisés dans les seringues : ce sont des pièces obtenues par moulage d'élastomères compatibles pour une longue durée avec le principe actif : chaque pièce intègre les fonctions de piston et d'étanchéité par la réalisation de bourrelets ou de lèvres (non détaillée sur les figures). Les élastomères habituellement utilisés pour la fabrication de ces pièces sont par exemple des chlorobutyl ou bromobutyl, dont la dureté Shore est réglée entre environ 45 et environ 70. Ces pièces peuvent recevoir des traitements de surface notamment pour faciliter leurs déplacements dans le réservoir tubulaire. Lorsqu'il est libre, le bouchon-piston a un diamètre supérieur d'environ 10 pour cent au diamètre intérieur du tube qui va le recevoir, la hauteur du bouchon-piston est d'environ 0,5 à 0,8 fois ce diamètre. Lorsque le bouchon-piston est engagé dans le tube, du fait des déformations, sa hauteur est égale à environ 0,6 fois à environ 1,0 fois le diamètre intérieur du réservoir.

Le réceptacle 7 est dans cet exemple, voir aussi figure 2, une pièce de forme extérieure cylindro-conique qui comporte un alésage central 10 dans lequel va venir se loger l'obturateur aval 5. Sur sa périphérie le réceptacle comporte trois conduits d'injection dont un seul, repéré 8, est visible sur cette coupe. Les conduits d'injection, tels que le conduit 8, traversent toute la hauteur du réceptacle 7 depuis la face amont jusqu'à la face aval. Ils communiquent avec l'alésage central 10 par des entrées 9 constituées par un lamage positionné sur le conduit d'injection et un canal radial reliant ledit lamage à l'alésage central 10. Le volume libre de l'alésage borgne 10 du réceptacle 7 est égal à celui de l'obturateur aval 5. Lorsque l'obturateur aval 5 a atteint le fond 7a du réceptacle, l'entrée 9 (côté réservoir 3) du conduit d'injection 8 est mise en communication avec le liquide 6 ; le liquide s'écoule avec une vitesse correspondant à la pression transmise par l'obturateur amont 4.

Dans cette réalisation le moyen moteur agit sur l'obturateur amont par l'intermédiaire d'un piston 11 de section efficace égale à celle de l'obturateur amont 4. Ce piston 11 est en contact avec l'obturateur amont 4 il n'y a donc pas d'effet de choc ou de bélier en début de fonctionnement. Ce piston 11 grâce à son système d'étanchéité empêche les gaz produits par la combustion du chargement 72 de venir en contact avec l'obturateur amont et donc d'éventuelles détériorations de celui-ci et des fuites de gaz vers le principe actif contenu dans le réservoir. Ce piston 11, d'une couleur adaptée, peut aussi servir d'indicateur de fonctionnement en apparaissant dans les fenêtres de visualisation du corps 2 de la seringue.

Nous allons décrire les principaux éléments, du générateur pyrotechniques 70. Il comprend dans le corps 71 au-dessus du piston un chargement pyrotechnique 72 dont la combustion est initiée par une amorce 73 impactée par un percuteur 74. L'amorce 73 est logée dans un porte-amorce. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76.

Le poussoir 78 coulisse sur l'extérieur du guide-percuteur 75 et il est retenu par des ergots 80 se déplaçant dans des rainures latérales 81. Ce poussoir 78 est ici l'organe de déclenchement.

Bien entendu pour initier la combustion du chargement pyrotechnique 72, sans sortir du cadre de l'invention, on peut utiliser des dispositifs d'initiation autre que le dispositif à percuteur ici décrit. Sans vouloir être exhaustif, nous citerons comme exemples des dispositifs d'initiation à pile électrique ou des dispositifs d'initiation piézo-électrique.

Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée.

Pour l'utilisation, après avoir enlevé le bouchon d'asepsie, et posé la face aval de l'injecteur sur la peau du sujet à traiter, l'opérateur appuie sur le poussoir 78 qui s'enfonce en comprimant le ressort 82. Le poussoir se déplace jusqu'à ce que la gorge 79 arrive à la hauteur de la gorge du percuteur 74, les billes, telle que la bille 77, retenant le percuteur 74, se dégagent dans la gorge 79 et libèrent le percuteur qui propulsé par le ressort 76 va impacter violemment l'amorce 73, dont l'initiation enflamme le chargement pyrotechnique 72. Le percuteur 74 en appui sur le porte-amorce 30 assure le maintien en place de l'amorce et l'étanchéité : les gaz de combustion ne remontant pas vers le poussoir. La combustion du chargement pyrotechnique va produire des gaz qui agissent sur le piston 11.

La figure 1 représente une seringue, selon l'invention, en forme de stylo : tous les éléments ont le même axe central mais sont superposés. Sans sortir du cadre de la présente invention d'autres dispositions sont envisageables par exemple la partie moteur peut faire un certain angle avec la partie réservoir-réceptacle pour arriver à des formes plus compactes comme cela est décrit par exemple dans la demande de brevet FR 2 815 544.

La figure 2 est un agrandissement de la partie aval de la seringue représentée sur la figure 1.

Pour le réceptacle 7 la striction par une protubérance circulaire de l'alésage 10 est réalisée par la superposition, tête-bêche, de deux troncs de cône. Un premier tronc de cône convergent, quand on s'y déplace axialement de l'amont vers l'aval : le diamètre d'entrée à l'amont de ce tronc de cône est égal au diamètre intérieur du réservoir 3, le diamètre final de ce tronc de cône est égal à environ 0,8 fois le diamètre d'entrée, la hauteur du tronc de cône est égale à environ 0,2 fois le diamètre d'entrée. Ce premier tronc de cône se raccorde, soit directement soit par une petite portion cylindrique à un tronc de cône divergent jusqu'à une partie de diamètre au plus égal à celui du réservoir 3 ; la hauteur de ce second tronc de cône peut occuper toute la hauteur restante de l'alésage 10 ou seulement une partie de cette hauteur, le reste étant une portion cylindrique se raccordant avec le fond 7a du réceptacle 7. Les raccordements entre les différentes parties sont préférentiellement arrondis pour éviter que des arêtes vives ne déchirent l'obturateur aval 5 quand il s'engage dans l'alésage et passe sur ces zones de raccordement.

La protubérance faisant striction telle que précédemment décrite, peut être fabriquée de différentes façons.

Par exemple par moulage direct du réceptacle avec un noyau déformable ou démontable, mais cette technique ne se **prête pas** très bien à une fabrication d'un grand nombre de pièces à grande cadence.

Préférentiellement le réceptacle est fabriqué en deux parties par moulage de deux pièces : l'une représentant le réceptacle mais avec un alésage central cylindrique et la seconde étant un insert bi-tronconique : les deux pièces sont ensuite assemblées par emmanchement forcé ou collage par toute méthode appropriée.

Pour les figures suivantes ne sera représentée, en perspective partiellement coupée, que le réceptacle de la seringue, sans entrer dans le détail du mode de montage dudit réceptacle dans le corps de la seringue. Par convention la face amont du réceptacle, telle que définie précédemment, sera celle dirigée vers haut de la page. Cette face amont comporte en général une gorge de joint circulaire ou quasi-circulaire qui reçoit un joint torique rapporté ou un joint bi-injecté si le réceptacle est fabriqué par injection. Sur la face avant chaque conduit d'injection est prolongé par une petite protubérance qui sert à l'appui du réceptacle sur la peau du patient. Nous ne reviendrons pas sur ces particularités dans chaque cas.

La figure 3 représente un exemple de réalisation d'un réceptacle 31 avec une seule protubérance circulaire fabriqué par l'assemblage de deux éléments. Un premier élément principal 32 qui constitue l'essentiel du réceptacle avec un alésage central 30 quasi cylindrique et les conduits périphériques 38 d'injection. Le deuxième élément 33 est un insert creux dont l'ouverture est bi-tronconique, avec une partie convergente depuis la face amont du réceptacle. Cet insert comporte des entailles radiales 39 coïncident avec entrées des conduits d'injection. Entre l'insert 32 et l'élément principal 31 se trouve aménagée une gorge circulaire qui va recevoir un joint d'étanchéité. L'insert emmanché dans l'élément principal vient en butée sur un épaulement de l'alésage. Dans cet exemple la protubérance n'occupe pas toute la hauteur de l'alésage borgne.

La figure 4 schématise un exemple de l'alésage central 40 d'un réceptacle 41 selon un second mode de réalisation de l'invention. L'alésage comprend superposées, les unes aux autres, plusieurs protubérances 42 constituées par une partie tronconique convergente et par un brusque élargissement jusqu'au diamètre d'ouverture de l'alésage ce qui donne un aspect en dent de scie à une section longitudinale de l'alésage.

La figure 5 représente de même un réceptacle 51 dont la protubérance 52 dans l'alésage central 50 est un taraudage jusqu'au fond de l'alésage : ici le taraudage est un filet de forme symétrique simple.

La figure 6 illustre un exemple de réceptacle selon une quatrième réalisation de l'invention. Le réceptacle 61 comporte un alésage central 60 légèrement tronconique et des conduits d'injection périphériques 68. Au droit de ces conduits d'injection on observe des saillies ovoïdes 62 sur la surface latérale de l'alésage : ces saillies sont essentiellement orientées selon les génératrices de la surface latérale de l'alésage. Ce sont des saillies, équiréparties comme les conduits d'injection qui font la striction de l'alésage. On note sur la face amont du réceptacle une gorge de joint quasicirculaire avec des lobes qui contournent les entrées 69 des conduits d'injection 68. Cette gorge de joint reçoit un joint multilobé ou préférentiellement le joint est injecté après la réalisation du réceptacle par une première injection. Cette technique de bi-injection est connue par ailleurs.

## Revendications

1. Seringue sans aiguille comportant un corps (2) logeant un réservoir cylindrique (3) fermé par un obturateur amont déplaçable (4) et un obturateur aval déplaçable (5) emprisonnant un principe actif (6) et comportant à l'aval un réceptacle (7,31,41,51,61) étant en appui sur le réservoir, ledit réceptacle comprenant un alésage central (10, 30, 40, 50, 60) et au moins un conduit périphérique d'injection (8, 38, 48, 58, 68) disposé autour de l'alésage central, ledit alésage central ayant une paroi latérale, l'obturateur aval (5) se logeant dans l'alésage central quand il est amené au contact du fond (7a) de l'alésage central dudit réceptacle par le fonctionnement d'un moyen moteur (70) déplaçant l'ensemble obturateur amont-liquide-obturateur aval, ladite seringue étant **caractérisée en ce que** ladite paroi latérale comporte au moins une protubérance qui fait striction par rapport à l'ouverture amont de l'alésage central et qui est configurée pour bloquer, dans l'alésage central, l'obturateur aval quand il y sera engagé, et **en ce que** le volume interne dudit alésage central permet le dégagement des entrées des conduits périphériques quand l'obturateur aval est logé dans l'alésage central.

2. Seringue sans aiguille selon la revendication 1 **caractérisée en ce que** l'alésage central (10) comporte une seule protubérance circulaire.

3. Seringue sans aiguille selon la revendication 2 **caractérisée en ce que** ladite protubérance circulaire est la superposition de deux troncs de cônes convergents puis divergent depuis la face amont du réceptacle.

4. Seringue sans aiguille selon la revendication 1 **caractérisée en ce que** l'alésage central comporte plusieurs protubérances circulaires superposées sur au moins une partie de la hauteur de l'alésage (10).

5. Seringue sans aiguille selon la revendication 4 **caractérisée en ce que** lesdites protubérances sont une superposition de plusieurs troncs de cônes convergents-divergents sur au moins une partie de la hauteur de l'alésage.

6. Seringue sans aiguille selon la revendication 1 **caractérisée en ce que** l'alésage central comporte une protubérance hélicoïdale en forme de taraudage (52).

7. Seringue sans aiguille selon la revendication 1 **caractérisée en ce que** l'alésage central comporte au moins une protubérance (62) le long d'une génératrice de l'alésage.

8. Seringue sans aiguille selon la revendication 7 **caractérisée en ce que** lesdites protubérances sont réparties au droit des conduits d'injection.

## Patentansprüche

1. Spritze ohne Nadel, umfassend einen Körper (2), in dem ein zylindrischer Behälter (3) aufgenommen ist, der von einem verschiebbaren vorgelagerten Verschluss (4) und einem verschiebbaren nachgelagerten Verschluss (5) verschlossen ist, der einen Wirkstoff (6) einschließt, und umfassend nachgelagert ein Gefäß (7, 31, 41, 51, 61), das auf dem Behälter aufliegt, wobei das Gefäß eine zentrale Bohrung (10, 30, 40, 50, 60) und mindestens eine periphere Injektionsleitung (8, 38, 48, 58, 68) umfasst wobei die zentrale Bohrung eine seitliche Wand aufweist, wobei der nachgelagerte Verschluss (5) in der zentralen Bohrung aufgenommen ist, wenn er in Kontakt mit dem Boden (7a) der zentralen Bohrung des Gefäßes durch den Betrieb eines Motormittels (70) gebracht wird, das die Einheit aus vorgelagertem Verschluss - Flüssigkeit - nachgelagertem Verschluss verschiebt, wobei die Spritze **dadurch gekennzeichnet ist, dass** die seitliche Wand mindestens einen Vorsprung umfasst, der mit Bezug auf die vorgelagerte Öffnung der zentralen Bohrung eine Einengung erzeugt, und der konfiguriert ist, um in der zentralen Bohrung den nachgelagerten Verschluss zu blockieren, wenn er darin eingegriffen ist, und dadurch, dass das innere Volumen der zentralen Bohrung die Freisetzung der Eingänge der peripheren Leitungen ermöglicht, wenn der nachgelagerte Verschluss in der zentralen Bohrung aufgenommen ist.

2. Spritze ohne Nadel nach Anspruch 1, **dadurch gekennzeichnet, dass** die zentrale Bohrung (10) einen einzigen ringförmigen Vorsprung umfasst.

3. Spritze ohne Nadel nach Anspruch 2, **dadurch gekennzeichnet, dass** der ringförmige Vorsprung die Überlagerung von zwei konvergierenden, dann divergierenden Kegelstümpfen ausgehend von der vorgelagerten Seite des Gefäßes ist.

4. Spritze ohne Nadel nach Anspruch 1, **dadurch gekennzeichnet, dass** die zentrale Bohrung mehrere ringförmige Vorsprünge umfasst, die auf mindestens einem Teil der Höhe der Bohrung (10) überlagert sind.

5. Spritze ohne Nadel nach Anspruch 4, **dadurch gekennzeichnet, dass** die Vorsprünge eine Überlagerung von mehreren konvergierenddivergierenden Kegelstümpfen auf mindestens einem Teil der Höhe der Bohrung sind.

6. Spritze ohne Nadel nach Anspruch 1, **dadurch gekennzeichnet, dass** die zentrale Bohrung einen schraubenförmigen Vorsprung in Form eines Gewindeschnitts (52) umfasst.

7. Spritze ohne Nadel nach Anspruch 1, **dadurch gekennzeichnet, dass** die zentrale Bohrung mindestens einen Vorsprung (62) entlang einer Vorrichtung zur Erzeugung der Bohrung umfasst.

8. Spritze ohne Nadel nach Anspruch 7, **dadurch gekennzeichnet, dass** die Vorsprünge gegenüber von den Injektionsleitungen verteilt sind.

## Claims

1. A needleless syringe including a body (2) housing a cylindrical reservoir (3) closed by a movable upstream shutter (4) and a movable downstream shutter (5) trapping an active ingredient (6) and including downstream a receptacle (7, 31, 41, 51, 61) bearing on the reservoir, said receptacle comprising a central bore (10, 30, 40, 50, 60) and at least one peripheral injection duct (8, 38, 48, 58 , 68) disposed around the central bore, said central bore having a side wall, the downstream shutter (5) being housed in the central bore when it is brought into contact with the bottom (7a) of the central bore of said receptacle by the operation of a drive means (70) moving the upstream shutter-liquid-downstream shutter set, said syringe being **characterized in that** said side wall includes at least one protrusion which is constricted relative to the upstream opening of the central bore and which is configured to block, in the central bore, the downstream shutter when it will be engaged therein, and **in that** the internal volume of said central bore allows clearing the inlets of the peripheral ducts when the downstream shutter is housed in the central bore.

2. The needleless syringe according to claim 1 **characterized in that** the central bore (10) includes only one circular protrusion.

3. The needleless syringe according to claim 2 **characterized in that** said circular protrusion is the superposition of two truncated cones converging then diverging from the upstream face of the receptacle.

4. The needleless syringe according to claim 1 **characterized in that** the central bore includes a plurality of circular protrusions superimposed on at least a portion of the height of the bore (10).

5. The needleless syringe according to claim 4 **characterized in that** said protrusions are a superposition of several convergent-divergent truncated cones on at least a portion of the height of the bore.

6. The needleless syringe according to claim 1 **characterized in that** the central bore includes a helical protrusion in the form of tapping (52).

7. The needleless syringe according to claim 1 **characterized in that** the central bore includes at least one protrusion (62) along a generatrix of the bore.

8. The needleless syringe according to claim 7 **characterized in that** said protrusions are distributed in line with the injection ducts.
